# EUROPEAN PATENT SPECIFICATION

(11) **EP 3 214 677 B1**
(45) Date of publication and mention of the grant of the patent: **28.08.2019**
(21) Application number: 15854150.8
(22) Date of filing: 27.10.2015
(51) Int. Cl.: H01M 4/66, H01G 11/28, H01M 4/86, H01M 8/18, H01G 11/68, H01G 11/70, H01M 4/04, H01M 4/62, C08F 14/18, C07D 207/267, H01M 10/0525

(54) **ELECTRODE CURRENT COLLECTOR, METHOD OF MANUFACTURING THE SAME, ELECTRODE, LITHIUM ION SECONDARY BATTERY, REDOX FLOW BATTERY AND ELECTRIC DOUBLE LAYER CAPACITOR**
ELEKTRODENSTROMKOLLEKTOR, VERFAHREN ZUR DESSEN HERSTELLUNG , ELEKTRODE, LITHIUM-IONEN-SEKUNDÄRBATTERIE, REDOX-DURCHFLUSSBATTERIE UND ELEKTRISCHER DOPPELSCHICHTKONDENSATOR
COLLECTEUR DE COURANT D'ÉLECTRODE, SON PROCÉDÉ DE FABRICATION, ÉLECTRODE, BATTERIE RECHARGEABLE AU LITHIUM-ION, BATTERIE REDOX ET CONDENSATEUR À DOUBLE COUCHE ÉLECTRIQUE

(30) Priority: 29.10.2014 JP 2014220565
(43) Date of publication of application: 06.09.2017
(73) Proprietor: Showa Denko K.K., Tokyo 105-8518 (JP)
(72) Inventor: SAITO, Shunsuke, Tokyo 105-8518 (JP); TAKEDA, Akifumi, Tokyo 105-8518 (JP); YOKOUCHI, Hitoshi, Tokyo 105-8518 (JP)
(74) Representative: Strehl Schübel-Hopf & Partner
(86) International application number: PCT/JP2015/080300
(87) International publication number: WO 2016/068156

(56) References cited:
- EP-A1- 2 738 853
- WO-A1-2013/018157
- WO-A1-2013/018159
- WO-A1-2014/171415
- JP-A- H10 255 808
- JP-A- 2001 284 184
- JP-A- 2002 231 589
- JP-A- 2005 191 423
- JP-A- 2012 124 133
- US-A1- 2005 103 229
- US-A1- 2007 109 722
- US-A1- 2008 113 253
- US-A1- 2013 196 230
- US-A1- 2014 162 122

## Description

### TECHNICAL FIELD

The present invention relates to an electrode current collector, a method of manufacturing an electrode current collector, an electrode, a lithium ion secondary battery, a redox flow battery, and an electric double layer capacitor.

### BACKGROUND ART

In recent years, lithium ion secondary batteries and redox flow batteries have attracted attention as secondary batteries. Lithium ion secondary batteries, in terms of light weight, small size, have been used as a power source of notebook computers, mobile phones, power tools, and the electronic and communications equipment. Recently, lithium ion secondary batteries are also used for electric vehicles and hybrid vehicles from the viewpoint of application to environmental vehicles. On the other hand, redox flow batteries are being put into practical use as 1000 kW class large power equipment from the viewpoint of high life cycle.

Electric double layer capacitors have also attracted a great deal of attention for the possibility of alternative of batteries because of the significantly higher storage amount.

A lithium ion secondary battery, a redox flow battery and an electric double layer capacitor have some partial structures similar to each other. An electrode is one of the similar structures. Regarding resistance of the electrode, as a common problem for each of the lithium ion secondary battery, the redox flow battery and the electric double layer capacitor, various studies have been conducted.

For example, a lithium ion secondary battery includes an electrode including a positive electrode using a metal oxide such as lithium cobalt oxide as a positive electrode active material and a negative electrode using a carbon material such as graphite as a negative electrode active material; and an electrolysis liquid using a carbonate as a solvent. In the lithium ion secondary battery, charging and discharging are performed by moving lithium ions between the positive electrode and the negative electrode.

The positive electrode is obtained by applying slurry composed of a positive electrode active material and a binder to a surface of a positive electrode current collector such as an aluminum foil, drying the coated slurry, and then cutting it into an appropriate size. The negative electrode is obtained by applying slurry comprising a negative electrode active material and a binder to a surface of a negative electrode current collector such as a copper foil, drying the coated slurry, and then cutting it into an appropriate size. For the positive electrode, an organic solvent-based slurry containing PVDF (polyvinylidene fluoride) or PTFE (polytetrafluoroethylene) as a binder is generally used. For the negative electrode, a water-based slurry containing SBR (styrene butadiene rubber) or an acrylic resin as a binder is generally used.

In recent years, due to increased demand for higher output and higher capacity, a process of etching a surface of a metal foil such as an aluminum foil which is used as a electrode current collector is performed. However, the electrode current collector obtained by the process has a problem of high cost, and it is impossible to sufficiently reduce the resistance value. In other words, it has a problem that it is impossible to obtain a suitable electrode current collector for rapid charging and discharging at low cost. The rapid charging / discharging characteristic means discharging performance and charging performance at a large current value, which is an important performance index of a secondary battery. Discharging performance is a characteristic that allows large current to flow. In order to output a large power, the performance becomes important. The charging performance is the ability to complete charging quickly with a large current. This performance is important in order to shorten the charging time and to make it efficient to use the secondary battery.

As a means for solving such a problem, an electrode current collector on which a coating layer containing carbon fine particles such as carbon is formed on a surface of a metal foil, has been proposed (Patent Documents 1 to 3). For the electrode current collector, since it is not necessary to process the metal foil, it can be inexpensively manufactured. Since the carbon particles in the coating layer contribute to electron conductivity of the electrode current collector, it is possible to realize a high electrical conductivity (a sufficiently low resistance). Resistance of the electrode current collector, as compared with the electrode current collector made of aluminum foil alone, can be decreased to 1/10 or less.

Further, in the secondary battery, in order to realize a further reduction in the resistance of the electrode, it has also been studied to miniaturize active material. When the active material is miniaturized, since the reaction area between the active material and the ions is increased, the resistance of the electrode can be decreased.

Other attempts have been made to uniformly form an active material on the surface of an electrode current collector in order to realize lower resistance of an electrode. In order to obtain an electrode in which an active material is uniformly formed on a surface of an electrode current collector, it is conceivable to increase wettability of the surface of the electrode current collector, that is, lower water repellency. Generally an electrode active material is uniformly formed by applying slurry containing a solvent to the surface of the electrode current collector. Since wettability of the current collector is increased, the slurry coating on the surface of the electrode is improved. As a result, the active material on the electrode can be uniformly formed. As described above, studies have been made to reduce water repellency of the surface of the electrode current collector to a low level; however, studies for increasing the water repellency have not been conducted.

### [Prior Art Document]

### [Patent document]

[Patent Document 1] Japanese Unexamined Patent Publication No. 2007-226969
[Patent Document 2] Japanese Unexamined Patent Publication No.
[Patent Document 3] Japanese Unexamined Patent Application Publication No. 2012-23050

Further relevant prior art can be found in: US 2014/162122 A1, US 2008/113253 A1, US 2005/103229 A1, 2013/196230 A1, US 2007/109722 A1, EP 2 738 853 A1.

### SUMMARY OF THE INVENTION

However, on an electrode current collector with lower water repellency, when attempting to fabricate an electrode forming a fine active material, problems have arisen in which it is impossible to obtain sufficient adhesion between the electrode current collector and the active material, and the electrode current collector is in danger of being damaged. As a result, it is impossible to make an electrode having sufficient performance.

This will be explained specifically using a positive electrode of a lithium ion secondary battery as an example, and the following reason is shown. When forming a positive electrode active material layer of a positive electrode, an organic solvent soluble binder is generally used as described above. For example, using slurry in which a positive electrode active material and a PVDF as a binder are dissolved in an organic solvent such as NMP (N-methylpyrrolidone), the positive electrode active material layer is formed on a metal foil such as aluminum foil. Since PVDF or the like used generally as a binder contains a bond of carbon (C) and fluorine (F), its surface energy is small. Therefore, if the positive electrode active material has a large particle size (15 µm or more) and has a large surface area, the positive electrode active material can be bonded sufficiently via a binder. In contrast, if the positive electrode active material has a small particle size (about 1 µm) and has small surface area, since it is impossible to obtain sufficient bonding, the positive electrode active material layer with sparse and dense portions is formed.

Therefore, as a binder having a relatively high surface energy, SBR (styrene butadiene rubber), acrylic resin or the like may be used. These materials have relatively high surface energy. Therefore, it is possible to bond, even when using a positive electrode active material with a small particle size.

In the case of preparing a positive electrode using these binders, it is common to coat a metal foil with slurry obtained by dissolving and dispersing the binders together with a positive electrode active material in an aqueous solvent.

However, since the positive active material contains lithium hydroxide (LiOH) and lithium carbonate (Li₂CO₃) as impurities, when the positive active material is dissolved or dispersed in an aqueous solvent, the solution becomes alkaline. Such a solvent having alkalinity corrodes a metal foil such as an aluminum foil which is an electrode current collector. When the metal foil is corroded, it is impossible to maintain a sufficient strength as an electrode. That is, although it is possible to realize a lower resistance of the positive electrode, since the strength decreases due to corrosion of the metal foil, the positive electrode produced using a water soluble binder has been hardly examined.

As described above, the positive electrode of the lithium ion secondary battery is described, but the same problems also occur in the negative electrode when LTO (lithium titanate) or the like is used as the negative electrode active material. Similar problems occur in redox flow batteries and electric double layer capacitors.

The present invention has been made in view of the above problems, and it is an object of the present invention to provide an electrode current collector, a method of manufacturing the same, an electrode, a lithium ion secondary battery, a redox flow battery, and an electric double layer capacitor, wherein the electrode current collector has sufficient adhesion between an electrode current collector and an active material, and can inhibit corrosion of a metal foil at low cost.

The present inventors have conducted intensive studies results, it was found that when a coating layer having a predetermined contact angle or more is formed on a metal foil, adhesion between an electrode current collector and active material is improved, and corrosion of the metal foil can be suppressed. Furthermore, the present invention provides an electrode current collector having a high electric conductive performance because that the electrode current collector includes a coating layer having electric conductivity. The present invention also provides an electrode, a lithium ion secondary battery, a redox flow battery and an electric double layer capacitor.

That is, the present invention provides means to solve the above-mentioned problems according to independent claim 1. Optional features are described in the dependent claims.

An electrode current collector according to an embodiment of the present invention includes a coating layer containing a water repellent material, which has the contact angle of 30° or more. Therefore, it is possible that an active material sufficiently adheres to the electrode current collector according to an embodiment of the present invention. Further it is possible to realize a high electric conductivity performance by forming a coating layer having a conductive assistant on the electrode current collector according to an embodiment of the present invention.

According to an embodiment of the present invention, a method of manufacturing an electrode current collector includes steps of applying a slurry having conductive assistant and the water repellent material suspended in a solvent to one or both surfaces of a metal foil, and drying coated slurry. Since a slurry in which the conductive assistant and the water repellent material are suspended is used, the layer formed by drying can achieve both high electric conductivity and water repellency. Further, it can be easily manufactured because it is possible to obtain a layer with sufficient functions as a layer after only drying.

The electrode of the present invention has the above-described electrode current collector. Therefore, it is possible to obtain sufficient adhesion between the electrode current collector and the active material.

The lithium ion secondary battery of the present invention has the electrode described above. Therefore, it has high electric conductivity and can reduce internal resistance.

The redox flow battery of the present invention has the electrode described above. Therefore, it has high electric conductivity and can reduce internal resistance.

The electric double layer capacitor of the present invention has the electrode described above. Therefore, it has high electric conductivity and can reduce internal resistance.

### BRIEF DESCRIPTION OF THE DRAWINGS

FIG. 1 is a schematic cross-sectional view of an electrode current collector according to one embodiment of the present invention.
FIG. 2 is an image obtained by photographing the upper surface of the coating layer with a scanning electron microscope (SEM).
FIG. 3 is a schematic cross-sectional view of an electrode according to one embodiment of the present invention.
FIG. 4 is a photograph of the surface of a positive electrode after being coated with a slurry containing a water soluble binder on a conventional electrode current collector and then dried.
FIG. 5 is a photograph of a surface of a positive electrode after being coated with a slurry containing a water soluble binder on an electrode current collector and then dried according to one embodiment of the present invention.
FIG. 6 is a schematic cross-sectional view of a lithium ion secondary battery according to one embodiment of the present invention.
FIG. 7 is a schematic cross-sectional view of a redox flow battery according to one embodiment of the present invention.
FIG. 8 is a schematic cross-sectional view of an electric double layer capacitor according to one embodiment of the present invention.

### DESCRIPTION OF THE INVENTION

Hereinafter, the electrode current collector, an electrode, a lithium ion secondary battery, a redox flow battery and an electric double layer capacitor according to an embodiment of the present invention will be described in detail with reference to drawings appropriately. In the drawings used in the following description, for the sake of easy understanding of the features of the present invention, there are cases where characteristic portions are enlarged for the sake of convenience, and the dimensional ratio of each component is different from the actual. Materials and sizes and the like in the following description are mere exemplary examples, and the present invention is not limited thereto, and it is possible to practice by modifying as appropriate within a range not changing the gist thereof, according to appended claims.

### [Electrode Current Collector]

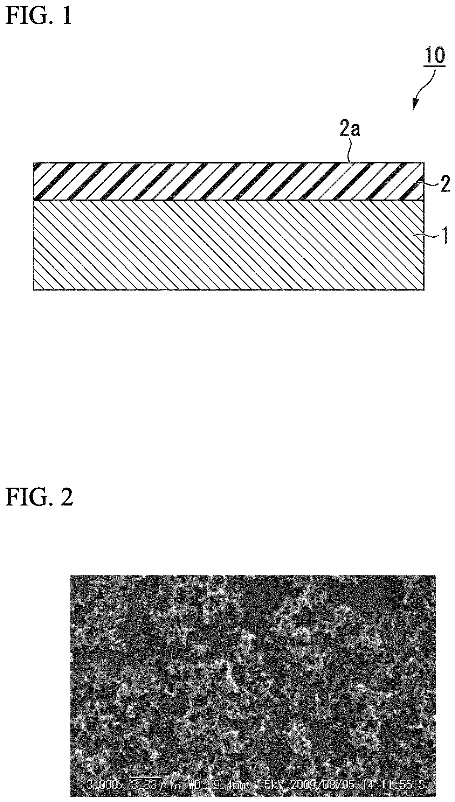

FIG. 1 is a schematic cross-sectional view of an electrode current collector according to one embodiment of the present invention. An electrode current collector 10 according to an embodiment of the present invention comprises a metal foil 1 wherein a coating layer 2 formed on one or both surfaces of the metal foil 1. The coating layer 2 includes a conductive assistant a resin and a fluorine-based polymer, the latter being a water repellent material. FIG. 1 shows a case where the coating layer 2 is formed on one surface of the metal foil 1, but the coating layer 2 may be formed on both surfaces, and the upper surface 2a is the surface of the coating layer 2 at a side not in contact with the metal foil 1.

FIG. 2 is an image obtained by photographing the upper surface of the coating layer with a scanning electron microscope (SEM). The part whitened on the image is the conductive assistant, and the conductive assistant is uniformly dispersed in the coating layer. The conductive assistant is coated with a resin to form a coating layer. Since the resin has no electric conductivity, it cannot be confirmed in FIG. 2, but when photographed using low accelerating voltage SEM or the like, it can be confirmed that a coating layer is formed on the entire surface of the metal foil (not shown). The water repellent material may be present dispersed in the coating layer, or it may function as a resin itself by being cured.

The coating layer 2 has a fluorine-based polymer, i.e. a water repellent material, and a contact angle with pure water of the upper surface 2a of the coating layer 2 is 30° or more. Here, pure water refers to distilled water or ion exchange water. In the case that the contact angle with pure water of the upper surface 2a of the coating layer 2 is at least 30°, when applying slurry containing for example a water soluble binder and a positive electrode active material on the upper surface 2a of the coating layer 2, water, the solvent used in binder, can be prevented from permeating into the coating layer toward the side of metal foil 1. When water can be prevented from permeating toward the metal foil 1 side, the following advantageous effects are exhibited.

Since a positive electrode active material of a lithium-ion battery contains lithium hydroxide (LiOH) and lithium carbonate (Li₂ CO₃) as impurity, when the material is dissolved or dispersed in an aqueous solvent, the solvent is alkalized. The solvent having such alkalinity corrodes the metal foil 1 when it comes into contact with the metal foil 1. However, when the contact angle with pure water of the upper surface 2a of the coating layer 2 is 30° or more, contact between the solvent and the metal foil can be avoided and corrosion of the metal foil can be suppressed. Such an effect is not limited to the electrode current collector for a positive electrode using a positive electrode active material, and a high effect can be exhibited also in a negative electrode in the case of using, for example, LTO (lithium titanate) or the like as a negative electrode active material.

Conventionally, studies were conducted to lower the contact angle and increase the wettability. This is because a more homogeneous positive electrode is formed by increasing the coatability of the slurry containing the positive electrode active material to be coated on the electrode current collector. For this reason, attempts to lower the contact angle and increase the wettability are generally studied in the field of batteries and the like, but on the contrary, attempts to increase the contact angle to increase the water repellency are hardly examined. The idea of increasing the water repellency is more difficult to think of for those skilled in the art.

The contact angle with pure water of the upper surface 2a of the coating layer 2 may be 30° or more, but it is preferably 60° or more, more preferably 100° or more. As the contact angle increases, contact between the metal foil and the solvent such as water can be avoided more. In addition, the contact angle with pure water cannot practically realize water repellency exceeding 170°, and it is preferably 120° or less. If the contact angle is too high, the coatability of the slurry to be coated on the coating layer 2 deteriorates.

The contact angle with pure water of the upper surface 2a of the coating layer 2 is obtained by the sessile drop method. Specifically, for example, a method of measuring 20 µl of pure water by dropping it on the upper surface 2a of the coating layer 2 in an ambient atmosphere at room temperature using an automatic contact angle measuring apparatus manufactured by Data Physics Co., Ltd. is used.

The coating layer 2 may contain at least a water repellent material and a conductive assistant. In the case where it is composed only of a water repellent material and a conductive assistant, this embodiment does not form part of the invention, a water repellent polymer may be used as a water repellent material, and a conductive assistant can be dispersed in the polymer. For example, when using a PVDF as a water repellent polymer and using carbon as a conductive assistant, slurry obtained by mixing these are applied on a metal foil, and thereafter the PVDF is crosslinked. The coating layer 2 formed in this method is a water repellent resin film (PVDF) in which a conductive assistant (carbon) is dispersed.

The coating layer 2 contains a water repellent material, a conductive assistant, and a resin. Here, the resin is not particularly limited as long as it is not fluorine-based polymer, i.e. a water repellent material, or a silicon-based polymer, a fluorine compound or a silicon compound, any resin may be used. For example, a resin compound obtained by polymerizing a polysaccharide polymer and a crosslinking agent can be used. As the polysaccharide polymer, chitosan, chitin and the like may be used. Examples of the crosslinking agent include acrylamide, acrylonitrile, chitosan pyrrolidone carboxylate, hydroxypropyl chitosan, 2-phosphonobutane-1,2,4-tricarboxylic acid, 3,3',4,4'-benzophenonetetracarboxylic acid, 2,3,6,7-naphthalenetetracarboxylic acid, 3,3',4,4'-biphenyltetracarboxylic acid, or acid anhydrides such as phthalic anhydride, maleic anhydride, trimellitic anhydride, pyromellitic anhydride, and so on may be used. In addition to the resin compound obtained by polymerizing the polysaccharide polymer and the crosslinking agent, polyacryl, polyolefin, polyamide, polyimide, polyamideimide, epoxy resin, bakelite, etc. may be used. Specific examples thereof include acrylic acid ester copolymers, polyacrylonitrile-based polymers, polypropylene oxide, polyethylene, polystyrene, polybutadiene, butyl rubber, nitrile rubber, SBR (styrene butadiene rubber), propylene-butadiene rubber, polysulfide rubber, nitrocellulose, cyanoethyl cellulose, various latex and the like.

The content of the water repellent material with respect to the total amount of the coating layer 2 is preferably 0.3% by mass to 90% by mass, more preferably 3% by mass to 67% by mass, and further preferably 13% by mass to 33% by mass.

When the content of the water repellent material with respect to the total amount of the coating layer 2 is 0.3% by mass or more, sufficient water repellency is exhibited, and it is confirmed that it is possible to suppress contact between the metal foil 1 and the solvent which is applied to the coating layer 2. That is, corrosion of the metal foil 1 can be further reduced. When the content of the water repellent material with respect to the total amount of the coating layer 2 is 90% by mass or less, adequate amounts of a conductive assistant and other resins are provided, and as a result, conductivity is maintained and high strength is realized.

The water repellent material is a fluorine-based polymer. Here, water repellency refers to a property of having low affinity with water and repelling water. As the water repellent material, a fluorine-based polymer is used. More specifically, fluorine-based polymers such as PVDF, PTFE, CTFE (polychlorotrifluoroethylene), HFE (hydrofluoroether), PFA (perfluoroalkoxyalkane), ETFE (ethylene tetrafluoroethylene copolymer), PVF (polyvinyl fluoride), VDF (vinylidene fluoride)-HFP (hexafluoropropylene) copolymer, VDF-HFP fluororubber, VDF-TFE (tetrafluoroethylene)-HFP fluororubber and the like, can be used.Examples, not forming part of the invention, are silicon-based polymers which can be used include silicones, such as oligomers or polymers obtained by dehydration condensation of silanol (R₃Si-OH), and the like. As the fluorine compound and silicon compounds, for example, an inorganic fluorine compound (Si-F) such as a silicon fluoride (SiF₄) or the like can be used.

When the water repellent material is a fluorine-based polymer, it is preferable to use a polymer in which at least a part thereof is acid-modified. The acid modification means that a newly added acid is added to the unsaturated bond portion of the fluorine-based polymer, wherein in the portion of the polymer, de-hydrofluoric acid reaction occurs. De-hydrofluoric acid may be performed, for example, by heating the fluorine-based polymer. Acid to be newly added is an acid such as an organic acid. The acid-modified fluoropolymer increases adhesiveness to the metal foil by added acid (for example, Japanese Patent No. 3966570). Since the molecular structure of the fluorine-based polymer is stable, that is, inactive, it also has the property that it is difficult to be bonded to other materials. Therefore, by partially denaturing the acid, adhesion to the metal foil can be enhanced. If adhesiveness between the metal foil 1 and the coating layer 2 is increased, current can be easily conducted from the metal foil 1 to the conductive assistant and the foil resistance can be reduced. That is, a low-resistance electrode current collector can be realized.

As the fluorine-based polymer, those described above can be used. As the acids and acid derivatives to be acid-modified, acrylic acid, methacrylic acid, methyl acrylate, ethyl acrylate, butyl acrylate, methyl methacrylate, ethyl methacrylate, butyl methacrylate, monomethyl maleate, monoethyl maleate, 2-carboxyethyl acrylate, 2-carboxyethyl methacrylate, acryloyloxyethyl succinic acid, methacryloyloxyethyl succinic acid, acryloyloxyethyl phthalic acid, methacryloyloxyethyl phthalic acid, trifluoroacrylic acid, trifluoromethylacrylic acid, 1,1-bis (acryloyloxymethyl) ethyl isocyanate, 2-acryloyloxyethyl isocyanate, 2-methacryloyloxyethyl isocyanate and the like can be used. For example, a fluorine-based polymer obtained by modifying a part of PVDF with monomethyl maleate, methyl acrylate, or methyl methacrylate; and a fluorine-based polymer obtained by modifying a part of PTFE with methyl acrylate or ethyl acrylate can be used.

The content of the conductive assistant in the coating layer 2 is preferably 23 mass% to 50 mass%, and more preferably 33 mass% to 50 mass%.

When the content of the conductive assistant in coating layer 2 is 23 mass% or more, it can exhibit sufficient electric conductivity. When the content of the conductive assistant is within the above range, it is also possible to increase the water repellency. Although the water repellent material is homogeneously dispersed in the coating layer 2, it is particularly likely to exist on the surface of the conductive assistant. Therefore, when the abundance ratio of the conductive assistant in the coating layer 2 is increased, the conductive assistant behaves as a foothold and the water repellency can be enhanced. When the content of the conductive assistant is 50% by mass or less with respect to the total weight of the coating layer 2 of the conductive assistant, the abundance ratio of the water repellent material in the coating layer 2 can be increased; as a result, high water repellency can be maintained.

The conductive assistant is not particularly limited as long as it serves to impart electrical conductivity and electrode stability (buffering function against volume change in the insertion and extraction of lithium ions) to the electrode. For example, fine carbon particles and carbon fibers such as carbon nanotubes and carbon nanofibers can be used. The carbon fibers are not particularly limited, but vapor grown carbon fibers are preferred. The carbon fine particles are not particularly limited, but acetylene black, Ketjen black, graphite (graphite) and the like are preferable. In particular, powder which has an electrical resistance of 1 × 10⁻¹ Ω·cm or less in a shape of 100% green compact is preferred, and a combination of the above materials may be used as necessary.

The particle size of the carbon fine particles used as the conductive assistant is not particularly limitted, but a particle size of approximately 10 to 100 nm is preferred. Regarding the shape, a shape having anisotropy such as needle-like or rod-like shape rather than a spherical shape is preferable. The reason, in the case of using the carbon fine particles as a conductive assistant of the lithium ion secondary battery, will be described below. Carbon fine particles with electron conductivity contribute to movement of electrons in a lithium ion secondary battery. During charging, electrons supplied from the exterior reaches the positive electrode active material via the aluminum foil. Therefore, it is preferable that the contact area between the aluminum foil and the positive electrode active material is large. Among the carbon fine particles, fine particles having a large surface area per mass are advantageous. Moreover, it is desirable that the amount of fine carbon particles is small in order to maintain the battery capacity. Therefore, carbon fine particles having anisotropic shapes are preferred.

It is preferable that a coating weight per unit area of the coating layer 2 on the metal foil 1 is 0.1g/m² to 10g/m² per one coated surface, and is more preferably 0.3 g/m² to 5 g/m². When the coating weight per unit area of the coating layer 2 is 0.1 g/m² or more, since the amount of water repellent material contained in the coating layer 2 is also increased, it is possible to enhance the water repellency. When the coating weight per unit area of the coating layer 2 is 10g/m² or less, the electrode current collector 10 can exhibit sufficient performance. Therefore, it is not desirable in terms of productivity for the coating weight per unit area of the coating layer to be more than 10g/m².

The metal foil 1 is not particularly limited as long as it has high electric conductivity. For example, aluminum foil is commonly used for the positive electrode of a lithium ion secondary battery. As the negative electrode, copper foil is generally used.

### [Method of manufacturing an electrode current collector]

A method of manufacturing an electrode current collector according to an embodiment of the present invention includes steps of applying a slurry obtained by suspending a conductive assistant, a resin and a fluorine-based polymer, i.e. water repellent material in a solvent on one or both surfaces of the metal foil, and drying the coated slurry. The conductive assistant, a resin and a fluorine-based polymer, i.e. water repellent materials are used as described above.

As the solvent, any of organic solvent-based solvents or aqueous solvents may be used. The organic solvent-based solvents include, but are not limited to, methanol, ethanol, isopropanol, hexane, acetone, NMP and the like. The solvents can be used singly or a combination of two or more can be used.

The conductive assistant and water repellent material do not need to be dissolved in the solvent and may be dispersed in a solvent. Therefore, when using an aqueous solvent, since the water repellent material is generally not dissolved, it is sufficient that the water repellent material is dispersed in a solvent to provide suspended slurry. It is preferable to use water as a solvent because environmental load is small and the slurry can be manufactured at a low cost.

The content of fluorine-based polymer with respect to the total amount of the slurry is preferably 0.5 mass% to 10 mass%, more preferably 0.5 mass% to 3 mass%. When the content of the water repellent material with respect to the total amount of the slurry is within the range, the formed coating layer shows high water repellency. In addition, the solvent of the slurry containing active material which is coated on the coating layer 2 and the metal foil is prevented from being contacted with each other. That is, it is possible to further limit the corrosion of the metal foil.

The content of the conductive assistant with respect to the total amount of the slurry is preferably 3 mass% to 10 mass%, and more preferably 5 mass% to 10 mass%. If the content of the conductive assistant with respect to the total amount of the slurry is within the range, the formed coating layer can exhibit sufficient electric conductivity. Although water repellent material is homogeneously dispersed in the coating layer, it is particularly likely to exist on the surface of the conductive assistant. Therefore, when the abundance ratio of the conductive assistant in the coating layer increases, the conductive assistant behaves as a foothold and the water repellency can be enhanced.

Such a slurry is coated on one or both surface of the metal foil. The coating method is not particularly limited, and it is possible to use a method such as gravure coating, die coating, bar coating, spin coating, and nip-coating.

The viscosity of the slurry at the temperature used in the coating thereof is preferably 50mPa·s to 1000mPa·s, more preferably 50mPa·s to 500mPa·s, and most preferably 50mPa·s to 200mPa·s. When the viscosity of the slurry is too high, coating the metal foil is difficult. In contrast, when the viscosity of the slurry is too low, it becomes difficult to form a sufficient thickness thereof on the metal foil.

The coated slurry is dried to form a coating layer. The coating layer can perform well even if it was only obtained by evaporating the solvent from the slurry. If the solvent is not sufficiently evaporated, it is difficult to maintain the high strength and water repellency of the coating layer; therefore, drying is preferably carried out at a higher temperature than the evaporation temperature of the solvent at atmospheric pressure.

As the slurry has a resin component, it is more preferable to cure the resin component. Regarding thermosetting resins, it is more preferable that the drying temperature is equal to or higher than the curing temperature (crosslinking reaction temperature) of the contained resin. The slurry may more preferably contain a catalyst to promote such a curing reaction, a polymerization agent, or a crosslinking agent.

### [Electrode]

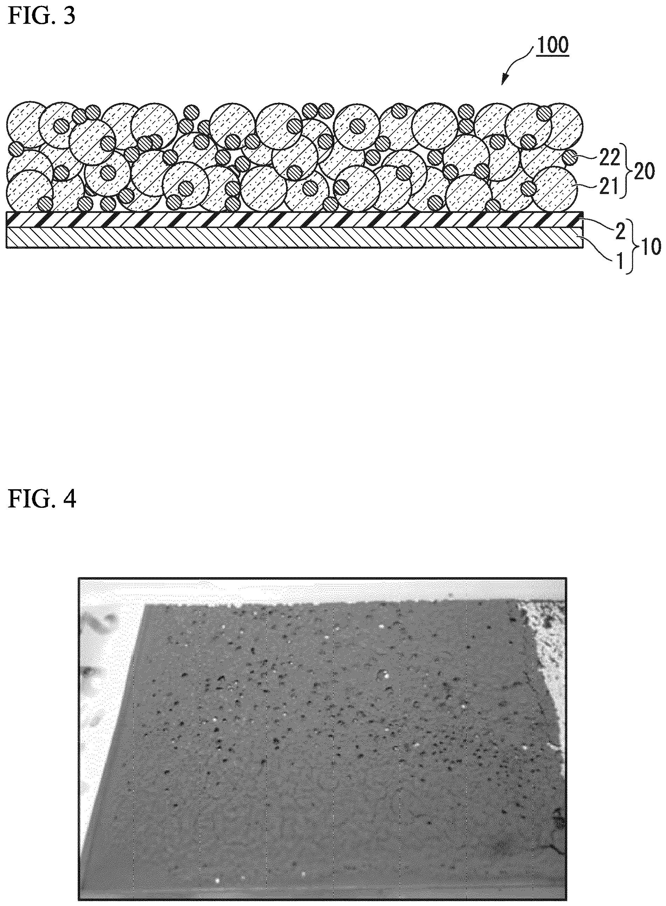

FIG. 3 is a schematic sectional view of an electrode according to an embodiment of the present invention. An electrode 100 according to an embodiment of the present invention includes an electrode current collector 10 as described above. FIG. 3 schematically shows an electrode for a positive electrode of a lithium ion secondary battery. On one surface of the electrode current collector 10, the positive active material layer 20 including the positive active material 21 and the positive electrode conductive assistant 22 which are bonded via a binder (not shown) are formed. Hereinafter, a positive electrode of a lithium ion secondary battery as an example of the electrodes will be explained.

Conventionally, a positive electrode is formed by applying a slurry to an electrode current collector wherein the slurry is formed by suspending a positive electrode active material, a conductive assistant for the positive electrode, and a binder in a solvent, and drying the coated slurry. As the binder, a PVDF or the like which can be dissolved in an organic solvent is generally used. This is because when aqueous slurry containing SBR or acrylic resin or the like is used, the metal foil is corroded due to contact between water and the metal foil. Figure 4 is a photograph of the surface of the positive electrode after the aqueous slurry coated on a conventional electrode collector is dried. As shown in FIG. 4, the surface of the positive electrode has a plurality of minute holes, and as a result, the electrode current collector is corroded.

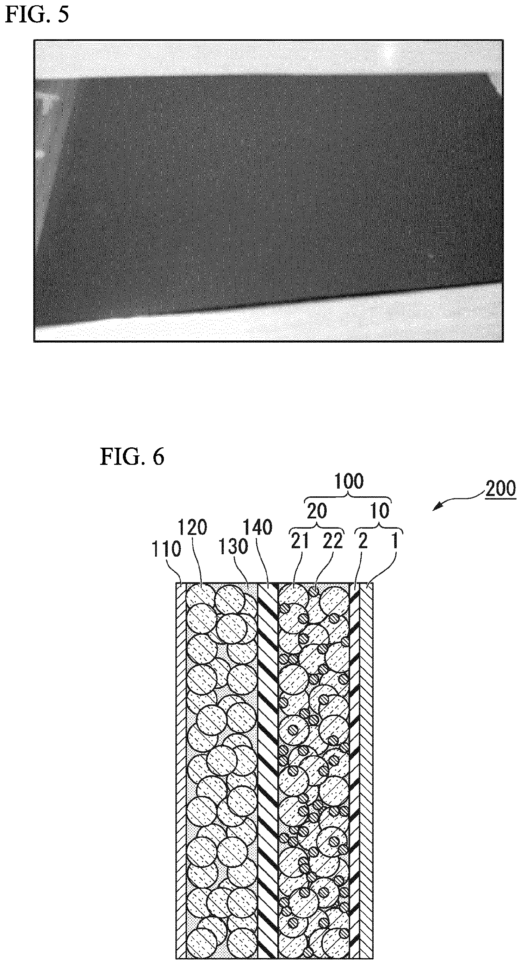

In contrast, the present invention according to one embodiment electrode 100 includes an electrode current collector 10 as described above. Since the surface of the electrode current collector 10 at a side of the positive electrode active material layer 20 is water repellent, it is possible to prevent a solvent of slurry containing an electrode active material from contacting a metal foil. The solvent of the slurry to be coated on the electrode current collector 10 is not limited to an organic solvent, and water may also be used. The binder is not limited to PVDF and the like of the organic solvent base, and a water-based binder, such as SBR and an acrylic resin-based emulsion, may be used. FIG. 5 is a photograph of the positive electrode of the surface after drying the aqueous slurry coated on the electrode current collector according one embodiment of the present invention. As shown in FIG. 5, even when water is used, the electrode current collector is not corroded. Therefore, it is possible to obtain the electrode 100 having high adhesion between the electrode current collector 10 and the positive electrode active material layer 20.

As the positive active material 21 and the positive electrode conductive assistant 22, materials generally used may be used.

As the positive electrode active material 21, for example, lithium cobalt oxide (LiCoO₂), lithium manganate (LiMn₂O₄), lithium nickel oxide (LiNiO₂), ternary lithium compounds of Co, Mn, Ni (Li(CoₓMn_{y}Ni_{z}) O₂), sulfur-based material (TiS₂), olivine material (LiFePO₄) and the like may be preferably used. As the positive electrode conductive assistant 22, for example, acetylene black, Ketjen black, vapor grown carbon fiber, graphite and the like may be preferably used.

The positive electrode of the lithium ion secondary battery is described as an example for the electrodes, but the present invention according to one embodiment the electrode is not limited to the positive electrode of a lithium ion secondary battery. For example, when a slurry containing a solvent, a negative electrode active material and a binder is applied to one or both surfaces of an electrode current collector used for a negative electrode of a lithium ion secondary battery, a layer containing the negative electrode active material and the binder is formed on one or both surfaces of the electrode current collector. For example, when the electrode is used for an electric double layer capacitor, a layer including a porous carbon material is formed on one or both surfaces of an electrode current collector.

### [Lithium ion secondary battery]

FIG 6 is a cross-sectional schematic view of a lithium ion secondary battery according to one embodiment of the present invention. The present invention according to one embodiment the lithium-ion secondary battery 200 includes the above-described electrode 100. The electrode 100 is a positive electrode (hereinafter in the description of the lithium ion secondary battery, the electrode 100 is described as a positive electrode 100) including the coating layer 2 formed on the metal foil 1 and a positive electrode active material layer 20 formed on the coating layer 2, wherein the positive electrode active material layer 20 contains a positive active material 21, a conductive assistant 22 and a binder. The lithium ion secondary battery 200 is obtained by bonding a positive electrode 100 and a negative electrode containing a negative electrode current collector 110 and the negative electrode active material 120 via a separator 140, and then filling an electrolyte 130 inside.

The lithium ion secondary battery 200 may be discharged by being connected to a load, such as a motor or a light source to the positive electrode 100 and negative electrode (not shown), and be charged by being connected to the power supply (not shown).

A lithium-ion secondary battery has a positive electrode 100 described above. Since the coating layer 2 of the positive electrode 100 partly contributes to the conductivity of the conductive metal foil 1, the resistance value as compared with the case where only the metal foil 1 is used may be made 1/10 or less. That is, the positive electrode 100 exhibits high conductivity. Therefore, the lithium ion secondary battery 200 is highly conductive, and as a result, it is possible to reduce internal resistance. Further, since the coating layer 2 has water repellency, corrosion of the metal foil 1 is inhibited when an aqueous solvent is used in the slurry. As a result, it is possible to obtain a lithium ion secondary battery having high adhesion between the electrode current collector 10 and the positive electrode active material layer 20.

As the negative electrode current collector 110, the negative active material 120, the electrolyte 130, and the separator 140, known materials may be used. As the negative electrode current collector 110, in addition to those known, the electrode current collector of the present invention may be used. As the negative electrode active material 120, for example, graphite, amorphous graphite, oxide and the like may be used. The electrolyte 130 is not limited to liquid, and a gel-like or solid electrolyte can be used. As the separator 140, for example, a film of polypropylene, polyethylene or the like may be suitably used.

An example of a lithium ion secondary battery, the electrode current collector according to one embodiment of the present invention is used in the positive electrode. In addition, the electrode current collector according to one embodiment of the present invention may be used only in the negative electrode. The electrode current collector according to one embodiment of the present invention may also be used in both of the positive electrode and the negative electrode. Although the lithium-ion secondary battery is described as a cell formed by bonding together the positive electrode and the negative electrode via a separator, it is possible to form a structure in which a plurality of cells are connected in series.

### [Redox flow battery]

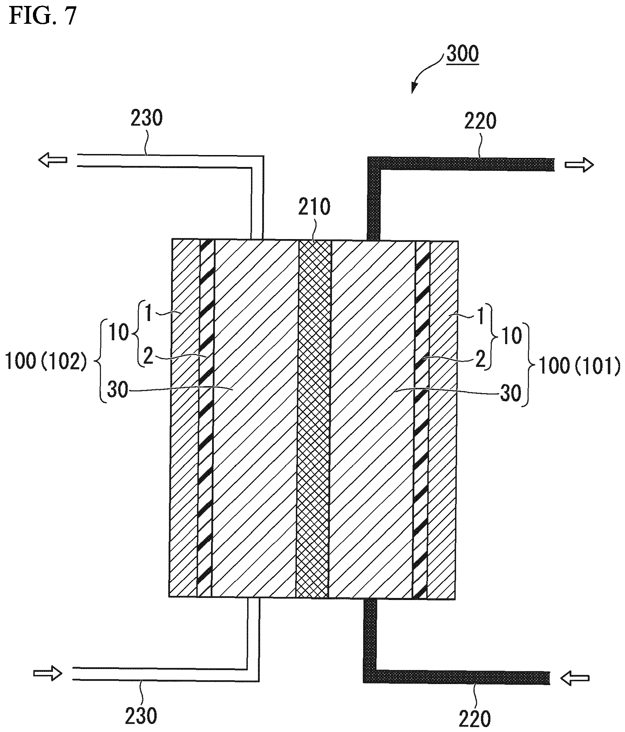

Figure 7 is a cross-sectional schematic view of a redox flow battery according to an embodiment of the present invention. Redox flow battery 300 of the present invention comprises the above electrode 100. In electrode 100, the coating layer 2 is formed on the metal foil 1, and a layer 30 comprising a porous carbon material is formed on the coating layer 2. The electrode 100 is formed on both surfaces of the separator 210, one of which functions as a positive electrode 101 and the other of which functions as a negative electrode 102. In the positive electrode 101, a positive electrode electrolyte is supplied through the positive electrode pipe 220, and in the negative electrode 102, and a negative electrode electrolyte is supplied through the negative electrode pipe 230.

The redox flow battery 300 may be discharged by being connected to the positive electrode 101 and the negative electrode 102 with loads such as motors and light source (not shown). The redox flow battery 300 may be charged by being connected to the power supply (not shown).

The positive electrode and the negative electrode have been described in redox flow cells formed as a single cell via a separator; however, it may have a structure in which a plurality of cells are connected in series. In this case, the positive electrode electrolytic solution is supplied from one tank to the positive electrodes of each cell, the negative electrode electrolyte is preferably configured to be supplieed from one tank to the negative electrodes of each cell. It is possible to flow electrolyte in the same state of charge to the respective batteries connected in series, and as a result, the state of charge of each battery can be equalized. In the battery having a structure which does not supply the electrolytic solution from the outside, it is impossible to achieve a uniform charge state, and as a result it is difficult to increase a scale, due to the individual difference of each battery and the installation environment. However, increasing a scale of the redox flow battery of the present invention can be easily realized by supplying the electrolytic solution to each battery in the same status from the same tank.

The redox flow battery 300 has the above-described electrode 100 (positive 101, negative electrode 102). Since the coating layer 2 of the electrode 100 contributes to conductivity of the conductive metal foil 1, the resistance value as compared with the case where only the metal foil 1 is used may be made 1/10 or less. That is, the positive electrode 100 exhibits high conductivity. Therefore, since the redox flow battery 300 has high conductivity, it is possible to reduce the internal resistance. Since the coating layer 2 has water repellency and corrosion of the metal foil 1 is inhibited, a redox flow battery having high adhesion between the layer 30 including a porous carbon material and the electrode current collector 10 can be obtained.

As the porous carbon material used in the layer 30 including a porous carbon material, known materials can be used. Further, as the separator 210, known materials can be used.

### [Electric double layer capacitor]

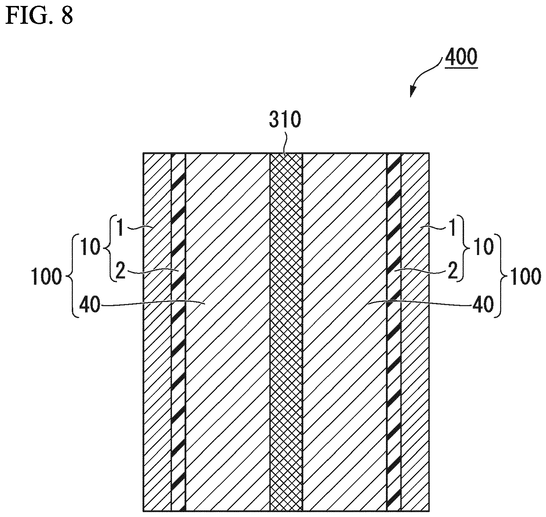

FIG 8 is a cross-sectional schematic view of an electric double layer capacitor according to one embodiment of the present invention. An electric double layer capacitor 400 of the present invention includes the above electrode 100. In the electrode 100, the coating layer 2 is formed on the metal foil 1, and a layer 40 containing the activated carbon is formed on the coating layer 2. Between the pair of opposing electrodes 100, the separator 310 is formed, in which an electrolyte is filled.

The electric double layer capacitor 400 may be discharged by being connected to a load (not shown) such as a motor and a light source with the pair of opposing electrodes 100. The electric double layer capacitor 400 may be charged by being connected to the power supply (not shown).

The electric double layer capacitor formed by bonding the two electrodes via a separator has been described as a single cell; however, it may have a structure in which a plurality of cells are connected in series.

Charging and discharging of the electric double layer capacitor 400 utilize the adsorption and desorption of ions into the electric double layer. The electric double layer is formed on the surface of activated carbon of the layer 40 containing the activated carbon by applying a voltage to the opposing electrodes 100, wherein the electrolysis of the electrolytic solution does not occur at the applied voltage. Ions are adsorbed by the electric double layer and the electric charge is stored. When the stored charge is discharged, the ions are desorbed from the electric double layer. Generally, the electric double layer capacitor 400 has an excellent charge-discharge life cycle.

Unlike batteries, since it do not involve a chemical reaction during charging and discharging, it is possible to realize an excellent charge-discharge life cycle.

The electric double layer capacitor 400 has the above-described electrode 100. Since the coating layer 2 of the electrode 100 contributes to conductivity of the conductive metal foil 1, the resistance value as compared with the case where only the metal foil 1 is used may be made 1/10 or less. That is, the electrode 100 may exhibit high electrical conductivity. Therefore, the electric double layer capacitor 400 is highly conductive. As a result, it is possible to reduce internal resistance. Further, since the coating layer 2 has water repellency, corrosion of the metal foil 1 can be inhibited. As a result, an electric double layer capacitor having high adhesion between an electrode current collector 10 and the layer 40 containing the active carbon can be obtained.

A preferred embodiment of the present invention has described in detail above; however, the various modifications and alterations of this invention will be apparent to those skilled in the art without departing from the scope and spirit of this invention.

### EXAMPLE

### [Examples 1-1 to 1-10]

In Examples 1-1 to 1-10, slurries containing various contents of a water repellent material were coated on a metal foil made of aluminum, and coating layers having a thickness of 1µm were formed by drying at a temperature of 190°C. The slurries used NMP as a solvent, in which acetylene black (HS-100) was used as a conductive assistant, a glyceryl chitosan and pyromellitic acid which were used to form a resin by crosslinking with one another, and a water repellent material such as PVDF (molecular weight 350,000) were dissolved in the solvent. The contents and viscosities of the slurries are described in Table 1. Examples 1-10 are different from the other Examples in that the slurry did not contain glyceryl chitosan and pyromellitic acid which were used to form a resin by crosslinking with one another in other Examples. Examples 1-6 to 1-10 are not forming part of the invention.

Then, a positive electrode active material layer was formed by coating a positive electrode slurry on the coating layer. The positive electrode slurry was obtained by suspending a positive electrode active material consisting of ternary lithium compounds of Co, Mn, Ni (Li(CoₓMn_{y}Ni_{z})O₂), a positive electrode conductive assistant consisting of acetylene black (HS-100), and a binder consisting of poly acrylate in water as a solvent, and the solution was alkaline. The composition ratios and performance of the formed coating layers, the appearance of the electrode current collector after forming a positive electrode active material layer are listed in Table 2.

The viscosity of the slurry was obtained by measuring the viscosity of the slurry before coating the surface of the metal foil. The viscosity was measured using No.2 spindle of B-type Rotary Viscometer under the circumferential speed of rotation of 60mm/s and at room temperature.

### Comparative Examples 1-1 to 1-2

Comparative Example 1-1 is different from Example 1-1 in the point that a coating layer did not contain water repellent material. Comparative Example 1-2 is different from Example 1-1 in the point that which no coating layer was formed. That is, in Comparative Example 1-2, a positive electrode active material layer was coated directly on a metal foil.

### [Table 1]

Acetylene black: Denki Kagaku Kogyo Co., Ltd.
Glyceryl chitosan: Dainichiseika Color & Chemicals Mfg. Co., Ltd.
Pyromellitic acid: Tokyo Chemical Industry Co., Ltd.
PVDF: Kureha Corporation
NMP: Mitsubishi Chemical Co., Ltd.

### [Table 2]

A NMP rubbing and water rubbing as shown in Table 2 are rubbing test results for confirming adhesion of the coating layers formed on the metal foils. Specifically, the rubbing test was carried out by rubbing the surface of the coating layer using a swab at one minute after dropping NMP or water on the coating layer formed on the metal foil. The presence or absence of peeling of the coating layer was examined. At this time, the load of the swab on the coating layer surface was 100 g. Results in which the coating layer was not peeled off after rubbing more than 10 times were expressed as "○", results in which the coating layer was peeled off at the stage when rubbing two to 10 times were expressed as "Δ", and results in which the coating layer was peeled off when rubbing one time were expressed as "×". When the electrode current collector is used for a lithium ion secondary battery or the like, such the operation of rubbing the surface is not carried out during practical use. Therefore, it can be determined to have sufficient abrasion resistance if the result is "Δ" or more. Further, it is more preferred that the result is "○", because it can withstand a physical impact during operation.

The foil resistance shows a resistance value of the electrode current collector measured by the below method. The resistance value was measured by the following procedure. First, the electrode current collector on which the coating layer was formed on the metal foil was cut to form two pieces having a size of 20mm width and 100 mm length. Each end of the two pieces of the electrode current collector was brought into contact with a contact area of 20mm × 20mm. The two contact surfaces faced each other in the manner such that both of the contact surfaces were on the coating layer side of the electrode current collectors. Then, the remained ends of the two electrode current collectors not in contact with each other were bonded to an AC milliohm meter to measure the penetration resistance. In Comparative Example 1-2, since no coating layer was formed, two electrode current collectors were brought into contact with each other at one of sides. When the foil resistance is 10Ω or less, the foil may function as a very good conductor having a low resistance value.

The contact angle was obtained by adding 20µl of water droplets on the coating layer of the electrode current collector, which was formed on the metal foil, and measured the contact angle using an automatic contact angle measuring device of the Data-Physics Co. under atmosphere and at room temperature.

The appearance of the electrode current collector was a result of evaluating the appearance of the electrode current collector by coating the positive electrode active material layer on the coating layer and drying the positive electrode active material layer. A result in which no change was observed in appearance was expressed as "○". A result in which when viewing from metal foil side of the electrode current collector, the surface of the coating layer did not be observed, however, the surface was roughened, was expressed as "Δ". The result that surface of the coating layer was observed when viewing from metal foil side of the electrode current collector, was expressed as "×". In other words, "×" indicates the electrode current collector does not function as an electrode current collector because the metal foil of the electrode current collector is corroded. "Δ" indicates that although the metal foil of the electrode current collector is not corroded, the electrode current collector functions as an electrode current collector.

As shown in Table 1 and Table 2, in Comparative Example 1-1 without a water repellent material and in Comparative Example 1-2 without a coating layer, the appearance after being coated with a positive active material layer was evaluated as "×", and the electrode current collectors did not function as an electrode current collector. In contrast, in Examples 1-3 to Example 1-10 having 3.3 mass% or more of water repellent material, the appearance after being coated with a positive electrode active material layer was evaluated as "○", and the electrode current collectors show high performance.

Examples 1-4 to 1-6 have high rubbing resistance, and the slurry viscosities are also within the range of 50 to 200mPa·s. Therefore, a water repellent material having a viscosity within the range is particularly preferred in terms of productivity.

In Example 1-10, PVDF as the water repellent material also functions as a cross-linked resin. Even in this case, it shows high water repellency and the appearance after being coated with the positive electrode active material was evaluated as "○". However, the coating layer was peeled off after being rubbed with water three times and being NMP rubbed four times. Therefore, it is more preferable to useanother resin.

In Comparative Example 1-2, the foil resistance was 30 Ω or more, and the electrode current collector had low conductivity. As compared with Examples 1-1 to 1-10 and Comparative Examples 1-1, it is possible to increase the conductivity of the electrode current collector by providing a coating layer having conductivity.

### [Examples 2-1 to 2-7]

In Examples 2-1 to 2-7, various contents and various types of conductive assistants were used. Other points were the same as in Example 1-5. The result for each slurry is shown in Table 3.The results of the formed coating layer are shown in Table 4. For reference, the results of Example 1-5 are listed again for reference.

### [Table 3]

Ketchen Black: Lion Co., Ltd.
VGCF (registered trademark) : Showa Denko KK

### [Table 4]

In Example 1-5 (acetylene black), Example 2-6 (Ketjen black), Example 2-7 (VGCF vapor grown carbon fiber), the same composition ratios of different types of conductive assistants were used, resulting in almost identical results. The results indicate that an electrode current collector having a good adhesion to the positive electrode active material layer and having a high conductivity can be obtained irrespective of the type of conductive assistants.

The larger the composition ratio of the conductive assistant used, the smaller the foil resistance and the higher conductivity obtained. In contrast, it is preferable that the composition ratio of conductive assistant is within a suitable range, so that good adhesion to the electrode current collector of the positive electrode active material layer is obtained.

### [Examples 3-1 to 3-5]

In Examples 3-1 to 3-5, PVDF was used as a water repellent material, and various types of PVDF with different molecular weights and in terms of the presence or absence of acid-modified portion were used. Other points were the same as in Example 1-5. The result of each slurry is shown in Table 5, and the results of the formed coating layers are shown in Table 6. For reference, the result of Example 1-5 is listed again.

### [Table 5]

### [Table 6]

In any one of Examples 3-1 to 3-5, the appearances after being coated with the positive active material layer were evaluated as "○". Good adhesion between the electrode current collector and the positive electrode active material layer was obtained. The result indicates that an electrode current collector having a good adhesion of the positive electrode active material layer and having a high conductivity can be obtained irrespective of the water repellent material.

Comparing Example 3-4 and Example 3-1, in which two PVDFs having the same molecular weight but in the presence or absence of the acid-modified portion are used, the result of Example 3-4 shows a higher contact angle. More PVDFs are bonded to the metal foil because the adhesion of PVDF to the metal foil increases after an acid modification treatment. The viscosity of the slurry increases when the molecular weight is more than a million.

### [Examples 4-1 to 4-5]

In Examples 4-1 to 4-5, various resins of coating layers were used. As the resins, polysaccharides polymer was fixed to glyceryl chitosan and various crosslinking agents were used. Other points were the same as in Example 1-5. The result of each slurry is shown in Table 7, and the results of the formed coating layers are shown in Table 8. For reference, the result of Example 1-5 is listed again.

### [Table 7]

Trimellitic acid: Tokyo Chemical Industry Co., Ltd.
2-phosphonobutane-1,2,4-tricarboxylic acid: Tokyo Chemical Industry Co., Ltd.
3,3',4,4'-benzophenonetetracarboxylic acid: Tokyo Chemical Industry Co., Ltd.
2,3,6,7-naphthalene tetracarboxylic acid: Tokyo Chemical Industry Co., Ltd.
3,3', 4,4'-biphenyltetracarboxylic acid: Tokyo Chemical Industry Co., Ltd.

### [Table 8]

In any one of Examples 4-1 to 4-5, the appearance after being coated with the positive active material layer is "o". Good adhesion between the electrode current collector and the positive electrode active material layer are obtained. The results indicate that an electrode current collector having a good adhesion of the positive electrode active material layer and having a high conductivity can be obtained irrespective of the resin of the coating layer.

### [Examples 5-1 to 5-7]

In Examples 5-1 to 5-7, various resins of the coating layer were used. As the solvent of the slurry for forming the coating layer, water was used instead of NMP. Other points were the same as in Example 1-5. The result of each slurry is shown in Table 9, and the results of the formed coating layer are shown in Table 10. For reference, the result of Example 1-5 is listed again. CMC (carboxymethylcellulose sodium salt) described in Example 5-4, for example, is a thickener, and is not a crosslink agent as an organic acid such as pyromellitic acid.

### [Table 9]

Acrylic acid ester copolymer: Showa Denko KK
CMC: Co., Daicel Co., Ltd
Polyacrylonitrile: INDIGO Inc.
Styrene-butadiene rubber: Nippon Zeon Co., Ltd.
PTFE: Daikin Industries, Ltd.

### [Table 10]

In Examples 5-1 to 5-7, in which water was used as a slurry solvent for forming the coating layer, the appearances after being coated with the positive active material layer are "Δ" or "○". The electrode current collector having a coating layer was formed. PVDF and PTFE as water repellent material in Examples 5-1 to 5-7 are insoluble in water. That is, a water repellent material is not necessarily dissolved in the slurry for forming the coating layer, and it is sufficient to disperse it. In any case, the coating layers after evaporation of the solvent show water repellency. It is possible to prevent the solvent, which is used in a step of forming the positive electrode active material layer, from contacting directly with the metal film.

### [Examples 6-1 to 6-6]

In Examples 6-1 to 6-6, various drying conditions for forming the coating layers were used. Other points were the same as in Example 1-5. The result of each slurry is shown in Table 11, and the results of the formed coating layer are shown in Table 12. For reference, the result of Example 1-5 is listed again.

### [Table 11]

### [Table 12]

In Examples 6-1 to 6-6, the appearances after being coated with the positive active material layer were "Δ" or "○". The electrode current collectors having a coating layer were formed. More stable films were formed when the drying temperature was 160°C or higher, which is the crosslinking temperature of the glyceryl chitosan and pyromellitic acid. For example, the results of NMP rubbing and water rubbing were "Δ" or "○", and sufficient abrasion resistance was obtained. Thus, it is possible to enhance the workability during operations. The appearance after being coated with the positive active material layer does not change and high adhesion is obtained. In contrast, when the drying temperature was more than 240°C, which approaches the heat limit temperature of the resin, various performances were poor.

### [Examples 7-1 to 7-6]

In Examples 7-1 to 7-6, various coating weights per unit area of the coating layers were used. Other points were the same as in Example 1-5. A coating weight per unit area is the weight of the coating layer per square meter. The composition ratios or the like of the slurry is shown in the table 13, and the evaluation results of the formed coating layers are shown in Table 14. For reference, the result of Example 1-5 and Comparative Example 1-2 are listed again.

### [Table 13]

### [Table 14]

As the coating weight per unit area of the coating layer is reduced, the appearance after being coated with the positive active material layer is deteriorated. The reduction of the coating weight per unit area of the coating layer means that the coating layer becomes thinner, or has a coarse-dense structure. Therefore, the effect of limiting the direct contact between the solvent and metal film when forming the positive electrode active material layer by providing a coating layer having water repellency is reduced.

### [Production of the electric double layer capacitor]

The electric double layer capacitor was prepared using the following three types of the metal foils.
Production Example 1. Aluminum foil on which the coating layer containing the water repellent material was formed (Example 1-5)
Production Example 2. Aluminum foil on which the coating layer containing no water repellent material was formed (Comparative Example 1-1)
Production Example 3. Aluminum foil on which no coating layer was formed

### (Comparative Example 1-2)

A layer containing activated carbon was formed using a slurry obtained by suspending 86 parts of alkali activated carbon (Kuraray Chemical Co., Ltd., YP-50F), 4 parts of acetylene black (Denki Kagaku Kogyo Co., Ltd., HS-100) as a conductive assistant, 9 part of polyacrylate(Showa Denko KK, Polysol (TM)) as a binder, and 1 part of carboxymethyl cellulose sodium (Daicel Co., Ltd.) in water as a solvent. Electrodes having the layer containing activated carbon and the above aluminum foil was used as electrodes of the electric double layer capacitor.

These electrodes were pressed at 10 tons roll press, and then were laminated via separators (Nippon Kodoshi Corporation Ltd., TF40). Electrode tabs and current collector portions of the laminates were ultrasonically welded together, and most potion of laminate materials were heat sealed except instilling portion. Injection was performed using 1M of propylene carbonate solution of triethylmethylammonium tetrafluoroborate (manufactured by Toyama Yakuhin Kogyo Co., Ltd.), and then it was sealed by a vacuum seal after impregnating the laminate. As a result, an electric double layer capacitor was prepared. The value of the equivalent series resistance (ESR) was obtained by measuring 1kHz AC impedance after the prepared cell was charged to 2.3V. The measurement results are shown in Table 15.

### [Table 15]

The result that the resistance value in Preparation Example 1 is the lowest one was obtained. This is because the formed coating layer contributes to conductivity of the conductive metal foil. Although Preparative Example 2 used a coating material, the resistance value is higher than the Production Example 1 containing water repellent material. The reason is shown below. In Production Example 2 containing no water repellent material, since glyceryl chitosan contained in the resin coating layer swells due to using pure water as solvent, electron conductivity of the coating layer is deteriorated. Preparation Example 3, in which no coating layer was formed, has the highest resistance value.

### Reference Signs List

1: Metal Foil,
2: Coating Layer,
2a: Upper Surface,
10: Electrode Current Collector,
21: Positive Electrode Active Material,
22: Electrode Conductive Assistant,
30: Layer Containing Porous Carbon Material,
40: Layer Containing Activated Carbon,
100: Electrode,
110: Negative Electrode Current Collector,
120: Negative Electrode Active Material,
130: Electrolyte,
140, 210, 310: Separator,
200: Lithium Ion Secondary Battery,
220: Pipe For Positive Electrode,
230: Pipe For Negative Electrode,
300: Redox Flow Battery,
400: Electric Double Layer Capacitor.

**Table 1**

| | Conductive assistant | Resin | | Water repellent Material | Solvent | Conductive assistant Content In Slurry | Polysaccharide Polymer Content In Slurry | The Crosslinking Agent Content In Slurry | Water repellent Material Content In Slurry | Slurry Viscosity |
|---|---|---|---|---|---|---|---|---|---|---|
| | | Polysaccharide Polymer | Crosslinking Agent | | | mass% | mass% | mass% | mass% | mPa·s |
| Example 1-1 | Acetylene Black HS-100 | Glyceryl Chitosan | Pyromellitic Acid | PVDF Molecular Weight 350,000 | NMP | 5 | 4.97 | 4.97 | 0.05 | 81 |
| Example 1-2 | | | | | | 5 | 4.97 | 4.97 | 0.1 | 83 |
| Examples 1-3 | | | | | | 5 | 4.75 | 4.75 | 0.5 | 84 |
| Examples 1-4 | | | | | | 5 | 4.5 | 4.5 | 1 | 88 |
| Examples 1-5 | | | | | | 5 | 4 | 4 | 2 | 93 |
| Examples 1-6 | | | | | | 5 | 3.5 | 3.5 | 3 | 150 |
| Examples 1-7 | | | | | | 5 | 2.5 | 2.5 | 5 | 410 |
| Examples 1-8 | | | | | | 5 | 1 | 1 | 8 | 640 |
| Examples 1-9 | | | | | | 5 | 0.5 | 0.5 | 9 | 730 |
| Examples 1-10 | | - | - | | | 5 | - | - | 10 | 820 |
| Comparative Example 1-1 | Acetylene Black HS-100 | Glyceryl Chitosan | Pyromellitic Acid | - | NMP | 5 | 5 | 5 | - | 80 |
| Comparative Example 1-2 | - | - | - | - | - | - | - | - | - | - |

**Table 2**

| | Conductive assistant Content In Coating Layer | Resin Content In Coating Layer | Water repellent Material Content In Coating layer | NMP Rubbing | Water Rubbing | Foil Resistance | Contact Angle | Appearance After Being Coated with Positive Electrode Active Material Layer |
|---|---|---|---|---|---|---|---|---|
| | mass% | mass% | mass% | Times | Times | Ω | Degree | |
| Example 1-1 | 33.3 | 66.4 | 0.3 | ○ | Δ | 0.13 | 65 | Δ |
| Example 1-2 | 33.3 | 66.0 | 0.7 | ○ | Δ | 0.13 | 92 | Δ |
| Examples 1-3 | 33.3 | 63.4 | 3.3 | ○ | ○ | 0.13 | 109 | ○ |
| Examples 1-4 | 33.3 | 60.0 | 6.7 | ○ | ○ | 0.15 | 111 | ○ |
| Examples 1-5 | 33.3 | 53.4 | 13.3 | ○ | ○ | 0.16 | 113 | ○ |
| Examples 1-6 | 33.3 | 43.4 | 23.3 | ○ | Δ | 0.23 | 115 | ○ |
| Examples 1-7 | 33.3 | 33.4 | 33.3 | Δ | Δ | 0.45 | 116 | ○ |
| Examples 1-8 | 33.3 | 13.4 | 53.3 | Δ | Δ | 0.73 | 116 | ○ |
| Examples 1-9 | 33.3 | 6.7 | 60.0 | Δ | Δ | 0.98 | 116 | ○ |
| Examples 1-10 | 33.3 | - | 66.7 | Δ | Δ | 1.2 | 117 | ○ |
| Comparative Example 1-1 | 33.3 | 66.7 | - | ○ | Δ | 0.2 | 21 | × |
| Comparative Example 1-2 | - | - | - | - | - | >30 | 29 | × |

**Table 3**

| | Conductive assistant | Resin | | Water repellent Material | Solvent | Conductive assistant Content In Slurry | Polysaccharide Polymer Content In Slurry | The Crosslinking Agent Content In Slurry | Water repellent Material Content In Slurry | Slurry Viscosity |
|---|---|---|---|---|---|---|---|---|---|---|
| | | Polysaccharide Polymer | Crosslinking Agent | | | mass% | mass% | mass% | mass% | mPa·s |
| Example 2-1 | Acetylene Black HS-100 | Glyceryl Chitosan | Pyromellitic Acid | PVDF Molecular Weight 350,000 | NMP | 0.5 | 4 | 4 | 2 | 43 |
| Example 2-2 | | | | | | 1 | 4 | 4 | 2 | 48 |
| Example 2-3 | | | | | | 3 | 4 | 4 | 2 | 75 |
| Examples 1-5 | | | | | | 5 | 4 | 4 | 2 | 93 |
| Examples 2-4 | | | | | | 10 | 4 | 4 | 2 | 180 |
| Examples 2-5 | | | | | | 20 | 4 | 4 | 2 | 520 |
| Examples 2-6 | Ketchen Black | | | | | 5 | 4 | 4 | 2 | 122 |
| Examples 2-7 | VGCF | | | | | 5 | 4 | 4 | 2 | 112 |

**Table 4**

| | Conductive assistant Content In Coating Layer | The Resin Content In Coating layer | Water repellent Material Content In Coating layer | NMP Rubbing | Water Rubbing | Foil Resistance | Contact Angle | Appearance After Being Coated with Positive Electrode Active Material Layer |
|---|---|---|---|---|---|---|---|---|
| | mass% | mass% | mass% | Times | Times | Ω | Degree | |
| Example 2-1 | 4.8 | 76.2 | 19.0 | ○ | ○ | 8.00 | 65 | Δ |
| Example 2-2 | 9.1 | 72.7 | 18.2 | ○ | ○ | 1.60 | 87 | Δ |
| Example 2-3 | 23.1 | 61.5 | 15.4 | ○ | ○ | 0.63 | 102 | ○ |
| Examples 1-5 | 33.3 | 53.3 | 13.3 | ○ | ○ | 0.16 | 113 | ○ |
| Examples 2-4 | 50.0 | 40.0 | 10.0 | ○ | Δ | 0.09 | 104 | ○ |
| Example 2-5 | 66.7 | 26.7 | 6.7 | Δ | Δ | 0.06 | 82 | Δ |
| Examples 2-6 | 33.3 | 53.3 | 13.3 | ○ | ○ | 0.11 | 117 | ○ |
| Examples 2-7 | 33.3 | 53.3 | 13.3 | Δ | ○ | 0.07 | 116 | ○ |

**Table 5**

| | Conductive assistant | Resin | | Water repellent Material (PVDF) | | Solvent | Conductive assistant Content In Slurry | Polysaccharide Polymer Content In Slurry | The Crosslinking Agent Content In Slurry | Water repellent Material Content In Slurry | Slurry Viscosity |
|---|---|---|---|---|---|---|---|---|---|---|---|
| | | Polysaccharide Polymer | Crosslinking Agent | Molecular Weight | Acid Denaturation | | mass% | mass% | mass% | mass% | mPa·s |
| Example 3-1 | Acetylene Black HS-100 | Glyceryl Chitosan | Pyromellitic Acid | 280,000 | None | NMP | 5 | 4 | 4 | 2 | 82 |
| Examples 1-5 | | | | 350,000 | None | | 5 | 4 | 4 | 2 | 93 |
| Example 3-2 | | | | 620,000 | None | | 5 | 4 | 4 | 2 | 150 |
| Example 3-3 | | | | 1,000,000 | None | | 5 | 4 | 4 | 2 | 420 |
| Example 3-4 | | | | 280,000 | 1 mass% | | 5 | 4 | 4 | 2 | 82 |
| Example 3-5 | | | | 1,000,000 | 0.3 mass% | | 5 | 4 | 4 | 2 | 460 |

**Table 6**

| | Conductive assistant Content In Coating Layer | Resin Content In Coating Layer | Water repellent Material Content In Coating layer | NMP Rubbing | Water Rubbing | Foil Resistance | Contact Angle | Appearance After Being Coated with Positive Electrode Active Material Layer |
|---|---|---|---|---|---|---|---|---|
| | mass% | mass% | mass% | Times | Times | Ω | Degree | |
| Example 3-1 | 33.3 | 53.4 | 13.3 | ○ | ○ | 0.15 | 112 | ○ |
| Examples 1-5 | 33.3 | 53.4 | 13.3 | ○ | ○ | 0.16 | 113 | ○ |
| Example 3-2 | 33.3 | 53.4 | 13.3 | ○ | ○ | 0.15 | 113 | ○ |
| Example 3-3 | 33.3 | 53.4 | 13.3 | ○ | ○ | 0.16 | 115 | ○ |
| Example 3-4 | 33.3 | 53.4 | 13.3 | ○ | Δ | 0.09 | 121 | ○ |
| Example 3-5 | 33.3 | 53.4 | 13.3 | Δ | Δ | 0.05 | 120 | ○ |

**Table 7**

| | Conductive assistant | Resin | | Water repellent Material | Solvent | Conductive assistant Content In Slurry | Polysaccharide Polymer Content In Slurry | The Crosslinking Agent Content In Slurry | Water repellent Material Content In Slurry | Slurry Viscosity |
|---|---|---|---|---|---|---|---|---|---|---|
| | | Polysaccharide Polymer | Crosslinking Agent | | | mass% | mass% | mass% | mass% | mPa·s |
| Examples 1-5 | Acetylene Black HS-100 | Glyceryl Chitosan | Pyromellitic Acid | PVDF Molecular Weight 350,000 | NMP | 5 | 4 | 4 | 2 | 93 |
| Example 4-1 | | | Trimellitic Acid | | | 5 | 4 | 4 | 2 | 89 |
| Example 4-2 | | | 2-Phosphonobutane-1,2,4-Tricarbanic Acid | | | 5 | 4 | 4 | 2 | 94 |
| Example 4-3 | | | 3,3',4,4'-Benzopheno netetracarboxylic Acid | | | 5 | 4 | 4 | 2 | 88 |
| Example 4-4 | | | 2,3,6,7-Naphthalenet etracarboxylic Acid | | | 5 | 4 | 4 | 2 | 92 |
| Example 4-5 | | | 3,3', 4,4'-Biphenyltetracar boxylic Acid | | | 5 | 4 | 4 | 2 | 95 |

**Table 8**

| | Conductive assistant Content In Coating Layer | Resin Content In Coating Layer | Water repellent Material Content In Coating layer | NMP Rubbing | Water Rubbing | Foil Resistance | Contact Angle | Appearance After Being Coated with Positive Electrode Active Material Layer |
|---|---|---|---|---|---|---|---|---|
| | mass% | mass% | mass% | Times | Times | Ω | Degree | |
| Examples 1-5 | 33.3 | 53.4 | 13.3 | ○ | ○ | 0.16 | 113 | ○ |
| Example 4-1 | 33.3 | 53.4 | 13.3 | ○ | ○ | 0.16 | 112 | ○ |
| Example 4-2 | 33.3 | 53.4 | 13.3 | ○ | ○ | 0.16 | 111 | ○ |
| Example 4-3 | 33.3 | 53.4 | 13.3 | ○ | ○ | 0.15 | 114 | ○ |
| Example 4-4 | 33.3 | 53.4 | 13.3 | ○ | ○ | 0.16 | 115 | ○ |
| Example 4-5 | 33.3 | 53.4 | 13.3 | ○ | ○ | 0.16 | 112 | ○ |

**Table 9**

| | Conductive assistant | Resin | Water repellent Material | Solvent | Conductive assistant Content In Slurry | Polysaccharide Polymer Content In Slurry | Water repellent Material Content In Slurry | Slurry Viscosity |
|---|---|---|---|---|---|---|---|---|
| | | | | | mass% | mass% | mass% | mPa·s |
| Examples 1-5 | Acetylene Black HS-100 | Glyceryl Chitosan / Pyromellitic Acid | Pvdf Molecular Weight 350,000 | NMP | 5 | 8 | 2 | 93 |
| Example 5-1 | | | | Water | 5 | 8 | 2 | 55 |
| Example 5-2 | | Glyceryl Chitosan / Trimellitic Acid | | | 5 | 8 | 2 | 46 |
| Example 5-3 | | Glyceryl Chitosan / 2-Phosphonobutane-1,2,4-Tricarbanic Acid | | | 5 | 8 | 2 | 49 |
| Example 5-4 | | Acrylic Acid Ester Copolymer / CMC | | | 5 | 8 | 2 | 96 |
| Examples 5-5 | | Polyacrylonitrile Type | | | 5 | 8 | 2 | 430 |
| Examples 5-6 | | Styrene Butadiene / CMC | | | 5 | 8 | 2 | 99 |
| Examples 5-7 | | Glyceryl Chitosan / Petromeric Acid | PTFF | | 5 | 8 | 2 | 82 |
| Comparative Example 5-1 | Acetylene Black HS-100 | Glyceryl Chitosan / Petromeric Acid | - | Water | 5 | 10 | - | 54 |
| Comparative Example 5-2 | | Glyceryl Chitosan / Petromeric Acid | | | 5 | 10 | - | 44 |
| Comparative Example 5-3 | | Glyceryl Chitosan / 2-Phosphonobutane-1,2,4-Tricarbanic Acid | | | 5 | 10 | - | 48 |
| Comparative Example 5-4 | | Acrylic Acid Ester Copolymer / CMC | | | 5 | 10 | - | 90 |
| Comparative Example 5-5 | | Polyacrylonitrile Type | | | 5 | 10 | - | 520 |
| Comparative Examples 5-6 | | Styrene Butadiene / CMC | | | 5 | 10 | - | 94 |

**Table 10**

| | Conductive assistant Content In Coating Layer | Resin Content In Coating Layer | Water repellent Material Content In Coating layer | NMP Rubbing | Water Rubbing | Foil Resistance | Contact Angle | Appearance After Being Coated with Positive Electrode Active Material Layer |
|---|---|---|---|---|---|---|---|---|
| | mass% | mass% | mass% | Times | Times | Ω | Degree | |
| Examples 1-5 | 33.3 | 53.4 | 13.3 | ○ | ○ | 0.12 | 113 | ○ |
| Example 5-1 | 33.3 | 53.4 | 13.3 | ○ | ○ | 0.14 | 112 | ○ |
| Example 5-2 | 33.3 | 53.4 | 13.3 | ○ | Δ | 0.13 | 110 | ○ |
| Example 5-3 | 33.3 | 53.4 | 13.3 | ○ | Δ | 0.14 | 113 | ○ |
| Example 5-4 | 33.3 | 53.4 | 13.3 | Δ | Δ | 0.18 | 109 | Δ |
| Examples 5-5 | 33.3 | 53.4 | 13.3 | ○ | Δ | 0.24 | 114 | ○ |
| Examples 5-6 | 33.3 | 53.4 | 13.3 | ○ | Δ | 0.22 | 108 | Δ |
| Examples 5-7 | 33.3 | 53.4 | 13.3 | ○ | ○ | 0.21 | 113 | ○ |
| Comparative Example 1-5 | 33.3 | 66.7 | - | ○ | Δ | 0.14 | 23 | × |
| Comparative Example 5-1 | 33.3 | 66.7 | - | ○ | Δ | 0.13 | 19 | × |
| Comparative Example 5-2 | 33.3 | 66.7 | - | ○ | Δ | 0.13 | 24 | × |
| Comparative Example 5-3 | 33.3 | 66.7 | - | Δ | × | 0.14 | 26 | × |
| Comparative Example 5-4 | 33.3 | 66.7 | - | ○ | Δ | 0.20 | 29 | × |
| Comparative Example 5-5 | 33.3 | 66.7 | - | Δ | × | 0.18 | 28 | × |

**Table 11**

| | Conductive assistant | Resin | | Water repellent Material | Solvent | Dry Temperature (°C) | Conductive assistant Content In Slurry | Polysaccharide Polymer Content In Slurry | The Crosslinking Agent Content In Slurry | Water repellent Material Content In Slurry |
|---|---|---|---|---|---|---|---|---|---|---|
| | | Polysaccharide Polymer | Crosslinking Agent | | | | mass% | mass% | mass% | mass% |
| Examples 6-1 | Acetylene Black HS-100 | Glyceryl Chitosan | Pyromellit ic Acid | PVDF Molecular Weight 350,000 | NMP | 120 | 5 | 4 | 4 | 2 |
| Example 6-2 | | | | | | 140 | 5 | 4 | 4 | 2 |
| Example 6-3 | | | | | | 160 | 5 | 4 | 4 | 2 |
| Example 1-5 | | | | | | 180 | 5 | 4 | 4 | 2 |
| Example 6-4 | | | | | | 200 | 5 | 4 | 4 | 2 |
| Example 6-5 | | | | | | 220 | 5 | 4 | 4 | 2 |
| Example 6-6 | | | | | | 240 | 20 | 4 | 4 | 2 |

**Table 12**

| | Conductive assistant Content In Coating Layer | Resin Content In Coating Layer | Water repellent Material Content In Coating layer | NMP Rubbing | Water Rubbing | Foil Resistance | Contact Angle | Appearance After Being Coated with Positive Electrode Active Material Layer |
|---|---|---|---|---|---|---|---|---|
| | mass% | mass% | mass% | Times | Times | Ω | Degree | |
| Example 6-1 | 33.3 | 53.4 | 13.3 | × | × | 0.76 | 45 | Δ |
| Example 6-2 | 33.3 | 53.4 | 13.3 | × | × | 0.68 | 48 | Δ |
| Example 6-3 | 33.3 | 53.4 | 13.3 | Δ | Δ | 0.18 | 96 | ○ |
| Example 1-5 | 33.3 | 53.4 | 13.3 | ○ | ○ | 0.16 | 113 | ○ |
| Example 6-4 | 33.3 | 53.4 | 13.3 | ○ | ○ | 0.14 | 115 | ○ |
| Example 6-5 | 33.3 | 53.4 | 13.3 | ○ | ○ | 0.15 | 112 | ○ |
| Examples 6 - 6 | 33.3 | 53.4 | 13.3 | Δ | Δ | 0.21 | 76 | Δ |

**Table 13**

| | Conductive assistant | Resin | | Water repellent Material | Solvent | Coating Weight Per Unit Area Of The Code Layer | Conductive assistant Content In Slurry | Polysaccharide Polymer Content In Slurry | Crosslinking Agent Content In Slurry | Water repellent Material Content In Slurry |
|---|---|---|---|---|---|---|---|---|---|---|
| | | Polysaccharide Polymer | Crosslinking Agent | | | g/m² | mass% | mass% | mass% | mass% |
| Example 7-1 | Acetyle ne Black HS-100 | Glyceryl Chitosan | Pyromel litic Acid | Pvdf Molecul ar Weight 350,000 | NMP | 0.05 | 5 | 4 | 4 | 2 |
| Example 7-2 | | | | | | 0.1 | 5 | 4 | 4 | 2 |
| Example 7-3 | | | | | | 0.3 | 5 | 4 | 4 | 2 |
| Example 1-5 | | | | | | 0.5 | 5 | 4 | 4 | 2 |
| Example 7-4 | | | | | | 1 | 5 | 4 | 4 | 2 |
| Example 7-5 | | | | | | 2 | 5 | 4 | 4 | 2 |
| Examples 7 -6 | | | | | | 5 | 5 | 4 | 4 | 2 |
| Comparative Example 1-2 | - | - | - | - | - | 0 | - | - | - | - |

**Table 14**

| | Conductive assistant Content In Coating Layer | Resin Content In Coating Layer | Water repellent Material Content In Coating layer | NMP Rubbing | Water Rubbing | Foil Resistance | Contact Angle | Appearance After Being Coated with Positive Electrode Active Material Layer |
|---|---|---|---|---|---|---|---|---|
| | mass% | mass% | mass% | Times | Times | Ω | Degree | |
| Example 7-1 | 33.3 | 53.4 | 13.3 | × | × | 6.50 | 45 | Δ |
| Example 7-2 | 33.3 | 53.4 | 13.3 | Δ | Δ | 1.55 | 92 | Δ |
| Example 7-3 | 33.3 | 53.4 | 13.3 | ○ | ○ | 018 | 110 | ○ |
| Examples 1-5 | 33.3 | 53.4 | 13.3 | ○ | ○ | 0.16 | 113 | ○ |
| Examples 7-4 | 33.3 | 53.4 | 13.3 | ○ | ○ | 0.14 | 117 | ○ |
| Example 7-5 | 33.3 | 53.4 | 13.3 | ○ | ○ | 0.17 | 119 | ○ |
| Examples 7-6 | 33.3 | 53.4 | 13.3 | ○ | ○ | 0.22 | 119 | ○ |
| Comparative Example 1-2 | - | - | - | - | - | > 30 | 29 | × |

**Table 15**

| | Conductive assistant | Resin | | Water repellent Material | Solvent | Conductive assistant Content Ratio In Coating Layer | Polysaccharide Polymer Content In Coating layer | Crosslinking Agent Content In Coating layer | Water repellent Material Content Ratio In Coating Layer | Equivalent Series Resistance (ESR) |
|---|---|---|---|---|---|---|---|---|---|---|
| | | Polysaccharide Polymer | Crosslinking Agent | | | mass% | mass% | mass% | mass% | mPa · S |
| Production Example 1 | Acetylene Black HS-100 | Glyceryl Chitosan | Pyromelliti c Acid Trimellitic Acid | Pvdf Molecu lar Weight 350,000 | NMP | 33.3 | 26.7 | 26.7 | 13.3 | 0.3 |
| Production Example 2 | | | | | | 33.3 | 33.3 | 33.3 | - | 0.5 |
| Production Example 3 | - | - | - | - | - | - | - | - | - | 5 |

## Claims

1. An electrode current collector comprising a metal foil,
wherein a coating layer is formed on one or both surfaces of the metal foil,
the coating layer comprises a conductive assistant, a fluorine-based polymer and a resin,
the resin is other than a fluorine-based polymer, a silicon-based polymer, a fluorine compound, a silicon compound, and
a content of the fluorine-based polymer with respect to a total amount of the fluorine-based polymer and the resin is 0.45 % to 20 % by mass.

2. The electrode current collector according to claim 1,
wherein a content of the fluorine-based polymer with respect to a total amount of the fluorine-based polymer and the resin is 5.0 % to 20 % by mass.

3. The electrode current collector according to claim 1 or 2,
wherein a content of the conductive assistant with respect to a total amount of the coating layer is 23% to 50% by mass.

4. The electrode current collector according to any one of claims 1 to 3,
wherein the fluorine-based polymer is acid-modified by adding an acid or an acid derivative at an unsaturated bond portion of the fluorine-based polymer,
the unsaturated bond portion is a portion of the polymer in which a de-hydrofluoric acid reaction occurs, and
the acid and the acid derivative is at least one compound selected from the group consisted of acrylic acid, methacrylic acid, methyl acrylate, ethyl acrylate, butyl acrylate, methyl methacrylate, ethyl methacrylate, butyl methacrylate, monomethyl maleate, monoethyl maleate, 2-carboxyethyl acrylate, 2-carboxyethyl methacrylate, acryloyloxyethyl succinic acid, methacryloyloxyethyl succinic acid, acryloyloxyethyl phthalic acid, methacryloyloxyethyl phthalic acid, trifluoroacrylic acid, trifluoromethylacrylic acid, 1,1-bis (acryloyloxymethyl) ethyl isocyanate, 2-acryloyloxyethyl isocyanate, and 2-methacryloyloxyethyl isocyanate.

5. The electrode current collector according to any one of claims 1 to 4,
wherein a coating weight per unit area of the coating layer is 0.1 g/m² to 10g / m² per one coated surface.

6. A method of manufacturing an electrode current collector according to any one of claims 1 to 5, comprising steps of:
applying a slurry obtained by suspending the conductive assistant and the fluorine-based polymer in a solvent on one or both surfaces of the metal foil, and
drying the slurry coated on the metal foil,
wherein the resin is other than a fluorine-based polymer, a silicon-based polymer, a fluorine compound, a silicon compound.

7. The method of manufacturing an electrode current collector according to claim 6.
wherein a content of the fluorine-based polymer with respect to a total amount of the slurry is 0.5% 10% by mass.

8. The method of manufacturing an electrode current collector according to claim 6 or 7.
wherein a content of the conductive assistant with respect to a total amount of the slurry is 3% to 10% by mass.

9. The method of manufacturing an electrode current collector according to any one of claims 6 to 8,
wherein the solvent is one of the group consisting of water and N- methylpyrrolidone.

10. An electrode comprising the electrode current collector according to any one of claims 1 to 5.

11. A lithium-ion secondary battery comprising the electrode according to claim 10.

12. A redox flow battery comprising the electrode according to claim 10.

13. An electric double layer capacitor comprising the electrode according to claim 10.

## Patentansprüche

1. Elektrodenstromabnehmer, umfassend eine Metallfolie,
wobei eine Beschichtungsschicht auf einer oder beiden Oberflächen der Metallfolie gebildet ist,
die Beschichtungsschicht einen leitenden Assistenten, ein Polymer auf Fluorbasis und ein Harz umfasst,
das Harz von einem Polymer auf Fluorbasis, einem Polymer auf Siliziumbasis, einer Fluorverbindung und einer Siliziumverbindung verschieden ist und
der auf eine Gesamtmenge des Polymers auf Fluorbasis und des Harzes bezogene Gehalt des Polymers auf Fluorbasis 0,45 bis 20 Masse-% beträgt.

2. Der Elektrodenstromabnehmer nach Anspruch 1, wobei ein Gehalt des Polymers auf Fluorbasis in Bezug auf eine Gesamtmenge des Polymers auf Fluorbasis und des Harzes 5,0 bis 20 Masse-% beträgt.

3. Der Elektrodenstromabnehmer nach Anspruch 1 oder 2, wobei ein Gehalt des leitenden Assistenten in Bezug auf eine Gesamtmenge der Beschichtungsschicht 23 bis 50 Gew.-% beträgt.

4. Der Elektrodenstromabnehmer nach einem der Ansprüche 1 bis 3,
wobei das Polymer auf Fluorbasis durch Zugabe einer Säure oder eines Säurederivats an einem ungesättigten Bindungsabschnitt des Polymers auf Fluorbasis säuremodifiziert ist,
der ungesättigte Bindungsabschnitt ein Abschnitt des Polymers ist, in dem eine Dehydrofluorsäure-Reaktion stattfindet, und
die Säure und das Säurederivat mindestens eine Verbindung ist, die aus der Gruppe ausgewählt ist, die aus Acrylsäure, Methacrylsäure, Methylacrylat, Ethylacrylat, Butylacrylat, Methylmethacrylat, Ethylmethacrylat, Butylmethacrylat, Monomethylmaleat, Monoethylmaleat, 2-Carboxyethylacrylat, 2-Carboxyethylmethacrylat, Acryloyloxyethylbernsteinsäure, Methacryloyloxyethylbernsteinsäure, Acryloyloxyethylphthalsäure, Methacryloyloxyethylphthalsäure, Trifluoracrylsäure, Trifluormethylacrylsäure, 1,1-Bis(acryloyloxymethyl)ethylisocyanat, 2-Acryloyloxyethylisocyanat und 2-Methacryloyloxyethylisocyanat besteht.

5. Der Elektrodenstromabnehmer nach einem der Ansprüche 1 bis 4, wobei ein Beschichtungsgewicht pro Flächeneinheit der Beschichtungsschicht 0,1 g/m² bis 10 g/m² pro einer beschichteten Oberfläche beträgt.

6. Verfahren zur Herstellung eines Elektrodenstromabnehmers nach einem der Ansprüche 1 bis 5, umfassend die Schritte von:
Auftragen einer Aufschlämmung, die durch Suspendieren des leitenden Assistenten und des Polymers auf Fluorbasis in einem Lösungsmittel auf eine oder beide Oberflächen der Metallfolie erhalten wurde, und
Trocknen der auf die Metallfolie aufgebrachten Aufschlämmung,
wobei das Harz von einem fluorbasierten Polymer, einem siliziumbasierten Polymer, einer Fluorverbindung, einer Siliziumverbindung verschieden ist.

7. Verfahren zur Herstellung eines Elektrodenstromabnehmers nach Anspruch 6, wobei ein Gehalt des Polymers auf Fluorbasis in Bezug auf eine Gesamtmenge der Aufschlämmung 0,5 Gew.-% beträgt.

8. Verfahren zur Herstellung eines Elektrodenstromabnehmers nach Anspruch 6 oder 7, wobei der auf die Gesamtmenge der Aufschlämmung bezogene Gehalt des leitfähigen Hilfsstoffs 3 bis 10 Gew.-% beträgt.

9. Verfahren zur Herstellung eines Elektrodenstromabnehmers nach einem der Ansprüche 6 bis 8, wobei das Lösungsmittel unter Wasser und N-Methylpyrrolidon ausgewählt ist.

10. Elektrode, umfassend den Elektrodenstromabnehmer nach einem der Ansprüche 1 bis 5.

11. Lithium-Ionen-Sekundärbatterie, umfassend die Elektrode nach Anspruch 10.

12. Redox-Fluss-Batterie, umfassend die Elektrode nach Anspruch 10.

13. Elektrischer Doppelschichtkondensator, umfassend die Elektrode nach Anspruch 10.

## Revendications

1. Collecteur de courant d'électrode comprenant une feuille métallique,
dans lequel une couche de revêtement est formée sur une ou les deux surface(s) de la feuille métallique,
la couche de revêtement comprend un assistant de conduction, un polymère à base de fluor et une résine,
la résine est autre qu'un polymère à base de fluor, un polymère à base de silicium, un composé de fluor et un composé de silicium, et
une teneur en polymère à base de fluor par rapport à une quantité totale du polymère à base de fluor et de la résine est de 0,45 % à 20 % en masse.

2. Collecteur de courant d'électrode selon la revendication 1, dans lequel une teneur en polymère à base de fluor par rapport à une quantité totale du polymère à base de fluor et de la résine est de 5,0 % à 20 % en masse.

3. Collecteur de courant d'électrode selon la revendication 1 ou 2, dans lequel une teneur de l'assistant de conduction par rapport à une quantité totale de la couche de revêtement est de 23 % à 50 % en masse.

4. Collecteur de courant d'électrode selon l'une quelconque des revendications 1 à 3,
dans lequel le polymère à base de fluor est modifié à l'acide en ajoutant un acide ou un dérivé d'acide à une partie de liaison insaturée du polymère à base de fluor,
la partie de liaison insaturée est une partie du polymère dans laquelle il se produit une réaction acide dé-hydrofluorique, et
l'acide et le dérivé d'acide sont au moins un composé sélectionné dans le groupe comprenant l'acide acrylique, l'acide méthacrylique, l'acrylate de méthyle, l'acrylate d'éthyle, l'acrylate de butyle, le méthacrylate de méthyle, le méthacrylate d'éthyle, le méthacrylate de butyle, le maléate de monométhyle, le maléate de monoéthyle, le 2-carboxyéthyle acrylate, le 2-carboxyéthyle méthacrylate, l'acide succinique d'acryloyloxyéthyle, l'acide succinique de méthacryloyloxyéthyle, l'acide phtalique d'acryloyloxyéthyle, l'acide phtalique de méthacryloyloxyéthyle, l'acide trifluoroacrylique, l'acide trifluorométhylacrylique, le 1,1-bis (acryloyloxyméthyle) éthyle isocyanate, le 2-acryloyloxyéthyle isocyanate et le 2-méthacryloyloxyéthyle isocyanate.

5. Collecteur de courant d'électrode selon l'une quelconque des revendications 1 à 4, dans lequel un poids de revêtement par unité de surface de la couche de revêtement est de 0,1 g/m² à 10 g/m² par surface revêtue.

6. Procédé de fabrication d'un collecteur de courant d'électrode selon l'une quelconque des revendications 1 à 5, comprenant les étapes suivantes:
appliquer une pâte obtenue par suspension de l'assistant de conduction et le polymère à base de fluor dans un solvant sur une ou les deux surface (s) de la feuille métallique, et
faire sécher la pâte déposée sur la feuille métallique,
dans lequel la résine est autre qu'un polymère à base de fluor, un polymère à base de silicium, un composé de fluor, un composé de silicium.

7. Procédé de fabrication d'un collecteur de courant d'électrode selon la revendication 6, dans lequel une teneur du polymère à base de fluor par rapport à une quantité totale de la pâte est de 0,5 % à 10 % en masse.

8. Procédé de fabrication d'un collecteur de courant d'électrode selon la revendication 6 ou 7, dans lequel une teneur de l'assistant de conduction par rapport à une quantité totale de la pâte est de 3 % à 10 % en masse.

9. Procédé de fabrication d'un collecteur de courant d'électrode selon l'une quelconque des revendications 6 à 8, dans lequel le solvant appartient au groupe comprenant l'eau et le N-méthylpyrrolidone.

10. Electrode comprenant le collecteur de courant d'électrode selon l'une quelconque des revendications 1 à 5.

11. Batterie secondaire lithium-ion comprenant l'électrode selon la revendication 10.

12. Batterie à flux redox comprenant l'électrode selon la revendication 10.

13. Condensateur électrique à double couche comprenant l'électrode selon la revendication 10.
